# EUROPÄISCHE PATENTSCHRIFT

(11) **EP 3 294 262 B1**
(45) Veröffentlichungstag und Bekanntmachung des Hinweises auf die Patenterteilung: **16.09.2020**
(21) Anmeldenummer: 16721435.2
(22) Anmeldetag: 10.05.2016
(51) Int. Cl.: A61K 8/34, A61K 8/37, A61Q 19/00

(54) **FLÜSSIGE KÜHLSTOFFZUBEREITUNGEN**
LIQUID COOLING COMPOSITIONS
COMPOSITIONS DES AGENTS REFROIDISSEMENT LIQUIDES

(30) Priorität: 15.05.2015 EP 15167910
(43) Veröffentlichungstag der Anmeldung: 21.03.2018
(73) Patentinhaber: Symrise AG, 37603 Holzminden (DE)
(72) Erfinder: DOSKOTZ, Eike, 50347 Erftstadt (DE); HIELSCHER, Thorsten, 37671 Höxter (DE); ENGELBRECHT, Andreas, 37603 Holzminden (DE); PÜTTCHER, Andrea, 37639 Bevern (DE); MACHINEK, Arnold, 37603 Holzminden (DE)
(74) Vertreter: Fabry, Bernd
(86) Internationale Anmeldenummer: PCT/EP2016/060474
(87) Internationale Veröffentlichungsnummer: WO 2016/184731

(56) Entgegenhaltungen:
- EP-A1- 2 457 554
- EP-A2- 1 332 772
- EP-A2- 2 033 688
- WO-A1-2007/128723
- WO-A2-2013/171018
- US-A- 3 023 253

## Beschreibung

### GEBIET DER ERFINDUNG

Die Erfindung befindet sich auf dem Gebiet der Kosmetik und betrifft Kühlstoffzubereitungen auf Basis von Menthol, die über einen weiten Temperaturbereich flüssig sind, kosmetische Produkte sowie insbesondere Kapseln, die diese enthalten, ihre Verwendung zur Herstellung von kosmetischen Produkten sowie ein Verfahren zur Kühlung von Haut und Schleimhäuten.

### STAND DER TECHNIK

Menthol ist ein natürlich vorkommender Wirkstoff, der beim Kontakt mit Schleimhäuten, speziell der Mundschleimhaut, einen kühlenden Effekt bewirkt. Menthol - und zahlreiche in der Folge entwickelte Mentholverbindungen mit teilweise deutlich gesteigerter Kühlwirkung - finden daher in Pharmazie, Kosmetik und Lebensmittelindustrie breite Anwendung. In natürlichen Quellen, beispielsweise dem Pfefferminzöl, kommt Menthol in Form von vier diastereomeren Enantiomerenpaaren vor, von denen nur die Hauptkomponente, das (-)-Menthol oder L-Menthol die gewünschten geschmacklichen und sonstigen sensorischen Eigenschaften hat, wie bereits **in** J. Am. Chem. Soc, Vol. 39 (8), 1917, S. 1515 bis 1525 beschrieben. So liegen insbesondere die Schmelzpunkte dieser verschiedenen Modifikationen zwischen 33 bis 43 °C wie in Archiv der Pharmazie, 307 (7), 1974, S. 497 bis 503 beschrieben. Der Schmelzpunkt der stabilen α- Modifikation liegt demgemäß bei 42 bis 43 °C.

Wegen dieser Lage der Schmelzpunkte kann L-Menthol sowie die meisten Mentholverbindungen an den Endverbraucher sowohl als in beheizten Behältern flüssig gehaltene Schmelze als auch in Form von Kristallen oder anderen erstarrten Formkörpern, wie Kompaktaten, Pastillen, Schuppen und dergleichen abgegeben werden. Generell weisen alle Feststoffe, die, wie L-Menthol und die dem Menthol strukturell verwandten Stoffe, einen Schmelzpunkt nur knapp oberhalb der Umgebungstemperatur haben, eine starke Neigung auf zu verbacken und zu verklumpen. Die Verarbeitung solchen nicht spezifikationsgerechten Materials ist aber mit erheblichem Zusatzaufwand verbunden. Soll nun reines L-Menthol bzw. Mentholverbindungen, d.h. nicht mit Hilfsmitteln wie z.B. Trennmitteln behandeltes Material als Feststoff verkauft werden, muss entweder durch eine geschlossene Kühlkette oder durch die Art der Formgebung sichergestellt werden, dass das Produkt den Endverbraucher in rieselfähiger Form erreicht.

Kommerziell ist beispielsweise Menthol in Form großer Kristalle verfügbar, die bei einer Länge von 0,5 bis 3 cm eine Dicke von 1 bis 3 mm aufweisen. Sie werden traditionell in kleinen Mengen aus natürlich gewonnenem Pfefferminzöl gezüchtet, in dem das Öl in Trögen oder Wannen über viele Tage in Kühlhäusern zur Kristallisation gebracht wird. Diese Kristalle weisen nur bei geringer Schütthöhe eine gute Rieselfähigkeit auf, verbacken aber zusehends bei erhöhter Last und/oder erhöhter Temperatur. Der technische Aufwand für die Kristallisation, Abtrennung und Reinigung der Kristalle und die geringe Raum-Zeit-Ausbeute eines solchen langwierigen Verfahrens machen es für die großtechnische Anwendung unattraktiv.

Bisherige Verfahren aus dem Stand der Technik versuchen, die Handhabung von Menthol mit Hilfe des Konfektionierungsverfahrens, speziell über die Partikelgröße zu verbessern.

### RELEVANTER STAND DER TECHNIK

Die DE 2530481 betrifft eine Vorrichtung zum Kristallisieren von Substanzen, insbesondere von optisch aktiven Mentholverbindungen, die unter Kristallisationsbedingungen grobe nadel- und balkenförmige Kristalle bilden. Das diskontinuierlich durchzuführende Kristallisationsverfahren wird unter Einsatz eines besonderen Rührwerkes durchgeführt, das ein Zusammenbacken der Kristalle in der Kristallsuspension verhindert. Das Wertprodukt wird abschließend durch eine Zentrifuge isoliert und in einem Trockner getrocknet.

Die beiden Patentschriften US 3,023,253 und US 3,064,311 beschreiben geschupptes L-Menthol sowie ein Verfahren zur Herstellung derartiger Schuppen durch Aufbringen einer Schmelze von L-Menthol auf einer gekühlten Tauchwalze. Falls gewünscht kann die Menthol-schmelze zwischen ein Paar gegenläufig rotierender, gekühlter Walzen eingebracht werden. Der auf der Tauchwalze ankristallisierte Mentholfilm wird nachbehandelt, indem er durch Wärmeeintrag getempert und durch Aufbringen zusätzlichen Menthols verstärkt wird. Beide Nachbehandlungen werden simultan mit einer Auftragswalze erreicht. Die so erhaltenen Schuppen weisen zunächst eine gute Rieselfähigkeit auf. Nach längerer Lagerung tritt jedoch eine leichte Verbackung ein, die eine mechanische Auflockerung durch Rütteln des Containers erforderlich macht. Es wird bemerkt, dass diese Verbackung durch eine zwar erwähnte, aber nicht näher charakterisierte poröse Oberfläche und damit einhergehende starke Sublimation des Produkts verursacht wird und dass das so erhaltene Produkt durch Kompaktieren zu Pellets weiterverarbeitet werden kann.

Das Prinzip der weiteren Vergröberung der Primärpartikel durch Kompaktierung wird auch in der DE 10224087 betreffend kompaktiertes Menthol in Form von Menthol- Presslingen sowie ein Verfahren zur Herstellung derselben beschrieben. Hier wird aber nicht auf die Wirkung der Partikelgröße allein abgehoben, sondern darauf, dass die Primärpartikel in einer spezifischen Kristallmodifikation vorliegen müssen. Durch Verpressen von Kristallen, die aus einer Lösungskristallisation oder einer Kühlwalzenverschuppung gewonnen wurden, lassen sich gegen Verbackung stabile Kompaktate erhalten, wenn diese überwiegend aus der thermodynamisch stabilen, erst bei 42,5 °C schmelzenden α-Modifikation bestehen.

Gegenstand der internationalen Patentanmeldung WO 2008 152009 A1 (BASF) ist ein Verfahren zur Herstellung von L-Menthol in fester Form durch Inkontaktbringen einer L-Menthol-Schmelze mit zwei voneinander beabstandeten gekühlten Oberflächen unter Erstarrung der L-Menthol-Schmelze zu L-Menthol in fester Form, wobei man den Kontakt zwischen der erstarrenden L-Menthol-Schmelze und den gekühlten Oberflächen mindestens bis zum Abschluss der Erstarrung aufrechterhält. Bei diesem Verfahren wird die Kristallisation des Menthols durch eine Kombination aus einem Vorkristallisierer und einem Doppelbandkühler bewirkt. Hierbei wird die Mentholsuspension in den Spalt zwischen zwei gekühlten Flächen gegeben und zum Erstarren bzw. Kristallisieren gebracht.

Die EP 2457554 A1 (SYMRISE) behandelt die Lager- und Temperaturproblematik von I-Menthol und d,l-Menthol im Bereich von 0 bis 30°C, die im genannten Temperaturbereichen als kristalline Feststoffe und nicht in flüssiger Form vorliegen. Als Lösung wird die Verwendung von d,l-Isomenthol in einem Gemisch mit Alkandiol(en) als Mittel zur Erniedrigung des Schmelzpunktes von d,l-Menthol, optional in Gegenwart von zusätzlichem l-Menthol, vorgeschlagen.

Gegenstand der WO 2013/171018 A2 (SYMRISE) ist eine Kühlmischung, umfassend ein Phenylalkenalderivat und einen weiteren Kühlwirkstoff sowie optional einen Aromastoff. Dabei werden Menthol bzw. l-Menthol als Kühlwirkstoffe und Menthylacetat als Aromastoff gelistet.

In der EP 2033688 A2 (SYMRISE) werden Oxalsäurederivate als physiologische Kühlwirkstoffe offenbart, wobei offenbart wird, dass die Mischung zusätzliche physiologische Kühlwirkstoffe, wie Menthol und Mentholderivate (I-Menthol, d-Menthol, Isomenthol, Menthylacetat usw.) enthalten kann.

### AUFGABE DER ERFINDUNG

Den Verfahren des Stands der Technik ist eine Reihe von ernsthaften Nachteilen gemeinsam, nämlich insbesondere eine geringe Lagerstabilität. Die Produkte beginnen unmittelbar nach der Lagerung zu verklumpen, es bilden sich Nadeln infolge Sublimation, die Kristalle brechen infolge unzureichender mechanischer Festigkeit, so dass in Summe letztlich unspezifikationsgemäße Ware resultiert, die zu mannigfaltigen Reklamationen Anlass gibt.

Die Aufgabe der vorliegenden Erfindung hat somit darin bestanden, Zubereitungen auf Mentholbasis zur Verfügung zu stellen, die frei von den eingangs geschilderten Nachteilen sind und insbesondere über einen weiten Temperaturbereich von 5 bis 20 °C flüssig vorliegen, sich bei Erstarren zumindest leicht wieder ohne Sublimation verflüssigen lassen und Menthol in seinen anwendungstechnischen Eigenschaften nicht nachstehen.

### BESCHREIBUNG DER ERFINDUNG

Gegenstand der Erfindung sind Kühlstoffzubereitungen, enthaltend
(a) L-Menthol,
(b) D-Menthol und
(c) mindestens einen weiteren Kühlstoff, ausgewählt aus der Gruppe, die gebildet wird von Isomenthol, Menthylacetat (L), Menthylacetat (D), Menthylacetat (iso), Essigsäurementhyllactat (iso) und Essigsäurementhyllactat (neo),
mit der Maßgabe, dass diese im Temperaturbereich von 5 bis 20 °C flüssig vorliegen.

Überraschenderweise wurde gefunden, dass der Zusatz der Kühlstoffe, die die Gruppe (c) bilden zu einer Mischung der beiden Mentholisomere eine Zubereitung ergibt, die auch in der Kälte flüssig ist und eine hohe Lagerstabilität bei 20 °C aufweist. Selbst Mischungen, die bei sehr niedrigen Temperaturen erstarren, lösen sich bei Umgebungstemperatur ohne Sublimationseffekte zu einer klaren Flüssigkeit auf. Ein weiterer Vorteil besteht darin, dass diese Mischungen dem klassischen Menthol weder in Geschmack, Geruch oder Kühlleistung nachstehen, sich jedoch wesentlich einfacher verarbeiten und insbesondere verkapseln lassen.

### KÜHLSTOFFZUBEREITUNGEN

Die erfindungsgemäßen Zubereitungen zeichnen sich insbesondere dadurch aus, dass sie
(a) 35 bis 55 Gew.-% L-Menthol,
(b) 25 bis 40 Gew.-% D-Menthol und
(c) 15 bis 30 Gew.-% Isomenthol, Menthylacetat (L), Menthylacetat (D), Menthylacetat (iso), Essigsäurementhyllactat (iso) und Essigsäurementhyllactat (neo) oder deren Gemische
mit der Maßgabe enthalten, dass sich die Mengenangaben jeweils zu 100 Gew.-% ergänzen.

Eine erste bevorzugte Zusammensetzung enthält
(a) L-Menthol,
(b) D-Menthol und
(c) Menthylacetat (L)
und zwar vorzugsweise:
(a) 50 bis 55 Gew.-% L-Menthol,
(b) 25 bis 35 Gew.-% D-Menthol und
(c) 18 bis 23 Gew.-% Menthylacetat (L)
mit der Maßgabe, dass sich die Mengenangaben jeweils zu 100 Gew.-% ergänzen.

Eine zweite bevorzugte Zusammensetzung enthält
(a) L-Menthol,
(b) D-Menthol,
(c1) Menthylacetat (L),
(c2) Menthylacetat (D),
(c3) Menthylacetat (iso) und
(c4) Essigsäurementhyllactat (neo, iso)
und zwar vorzugsweise:
(a) 50 bis 55 Gew.-% L-Menthol,
(b) 25 bis 35 Gew.-% D-Menthol,
(c1) 8 bis 12 Gew.-% Menthylacetat (L),
(c2) 8 bis 12 Gew.-% Menthylacetat (D),
(c3) 0,1 bis 1 Gew.-% Menthylacetat (iso) und
(c4) 0,1 bis 1 Gew.-% Essigsäurementhyllactat (neo, iso)
mit der Maßgabe, dass sich die Mengenangaben jeweils zu 100 Gew.-% ergänzen.

Eine dritte bevorzugte Zusammensetzung enthält
(a) L-Menthol,
(b) D-Menthol,
(c1) iso-Menthol und
(c2) Menthylacetat (L)
und zwar vorzugsweise:
(a) 35 bis 45 Gew.-% L-Menthol,
(b) 35 bis 45 Gew.-% D-Menthol,
(c1) 2 bis 8 Gew.-% iso-Menthol und
(c2) 15 bis 25 Gew.-% Menthylacetat (L)
mit der Maßgabe enthalten, dass sich die Mengenangaben jeweils zu 100 Gew.-% ergänzen.

Eine vierte bevorzugte Zusammensetzung enthält
(a) L-Menthol,
(b) D-Menthol,
(c1) iso-Menthol,
(c2) Menthylacetat (L),
(c3) Menthylacetat (D),
(c4) Menthylacetat (iso) und
(c5) Essigsäurementhyllactat (neo, iso)
und zwar vorzugsweise:
(a) 35 bis 45 Gew.-% L-Menthol,
(b) 35 bis 45 Gew.-% D-Menthol,
(c1) 2 bis 8 Gew.-% iso-Menthol,
(c2) 8 bis 12 Gew.-% Menthylacetat (L),
(c3) 8 bis 12 Gew.-% Menthylacetat (D),
(c4) 0,1 bis 1 Gew.-% Menthylacetat (iso) und
(c5) 0,1 bis 1 Gew.-% Essigsäurementhyllactat (neo, iso)
mit der Maßgabe, dass sich die Mengenangaben jeweils zu 100 Gew.-% ergänzen.

### KOSMETISCHE, DERMATOLOGISCHE UND/ODER PHARMAZEUTISCHE ZUBEREITUNGEN

Ein weiterer Aspekt der vorliegenden Erfindung betrifft kosmetische und/oder dermatologische Zubereitungen, die die oben genannten flüssigen Kühlstoffmischungen enthalten und zwar vorzugsweise in Mengen von 0,1 bis 10 Gew.-%, insbesondere 0,5 bis 8 Gew.-% und insbesondere 1 bis 5 Gew.-%. Bei den Mitteln kann es sich sowohl um Haut-, Körperpflege- oder Haarbehandlungsmittel, einschließlich Sonnenschutzmitteln und Mund- und Zahnpflegemitteln sowie (medizinischen) Kaugummis handeln. Besonders bevorzugte Anwendungen im Bereich der kosmetischen und dermatologischen Zubereitungen sind Duschbäder, Shampoos, Seifen, Lufterfrischer und dergleichen.

Zu den besonders bevorzugten pharmazeutischen Zubereitungen zählen Stoffe zur Linderung von Schmerzen der Schleimhäute, speziell Erkältungssäfte, -sprays, -dragees und - bonbons.

Die erfindungsgemäßen kosmetischen, dermatologischen und/oder pharmazeutischen Mittel können weitere typische Hilfs- und Zusatzstoffe enthalten, wie beispielsweise milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, UV-Lichtschutzfaktoren, Feuchthaltemittel, biogene Wirkstoffe, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### TENSIDE

Als oberflächenaktive Stoffe können anionische, nichtionische, kationische und/oder amphotere bzw. zwitterionische Tenside enthalten sein, deren Anteil an den Mitteln üblicherweise bei 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### ÖLKÖRPER

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### EMULGATOREN

Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich oder Cosmedia® SP von Cognis;
- Polyalkylenglycole sowie
- Glycerincarbonat.

Im Folgenden werden besonders geeignete Emulgatoren näher erläutert:
**Alkoxylate.** Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.
**Alkyl- und/oder Alkenyloligoglykosid.** Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise 8 geeignet sind. Der Oligomerisierungsgrad ist dabei ein statistischer Mittelwert, dem eine für solche technischen Produkte übliche Homologenverteilung zugrunde liegt.
**Partialglyceride.** Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.
**Sorbitanester.** Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.
**Polyglycerinester.** Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.
**Anionische Emulgatoren.** Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.
**Amphotere und kationische Emulgatoren.** Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### FETTE UND WACHSE

Typische Beispiele für Fette sind Glyceride, d.h. feste oder flüssige pflanzliche oder tierische Produkte, die im Wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### PERLGLANZWACHSE

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### ZUSÄTZLICHE KÜHLSTOFFE

Kühlstoffe sind Verbindungen, die auf der Haut ein Gefühlt der Kälte erzeugen. In der Regel handelt es sich dabei um Mentholverbindungen, die ausgewählt sind aus der Gruppe, die gebildet wird von Menthol Methyl Ether, Menthone Glyceryl Acetal (FEMA GRAS¹ 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.
¹ FEMA steht für "Flavor and Extracts Manufacturers Association" und GRAS ist definiert als "Generally Regarded As Safe". Eine FEMA GRAS Bezeichnung bedeutet, dass die so gekennzeichnete Substanz nach Standardmethode getestet und für toxikologisch unbedenklich erachtet wird.

Ein erster wichtiger Vertreter dieser Stoffe stellt das Monomenthyl Succinate (FEMA GRAS 3810) dar. Sowohl das Succinat als auch das analoge Monomenthyl Glutarate (FEMA GRAS 4006) stellen wichtige Vertreter von Monomenthylestern auf Basis von Di- und Polycarbonsäuren dar:Beispiele für Anwendungen dieser Stoffe finden sich in den Druckschriften WO 2003 043431 (Unilever) oder EP 1332772 A1 (IFF).

Die nächste wichtige Gruppe von im Sinne der Erfindung bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat® MGC), Menthol Propylenglycol Carbonate (FEMA GRAS 3784 = Frescolat® MPC), Menthol 2-Methyl-1,2-propandiol Carbonate (FEMA GRAS 3849) oder den entsprechenden Zuckerderivaten. Ebenfalls bevorzugt sind die Mentholverbindungen Menthyl Lactate (FEMA GRAS 3748 = Frescolat® ML) und insbesondere das Menthone Glyceryl Acetal (FEMA GRAS 3807) bzw. Menthone Glyceryl Ketal (FEMA GRAS 3808), das unter der Bezeichnung Frescolat® MGA vermarktet wird. Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonatw erwiesen, die die Anmelderin unter den Bezeichnungen Frescolat® MGA, Frescolat® ML, Frecolat® MGC und Frescolat® MPC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die bevorzugten Spezies WS-3, WS-4, WS-5, WS-12, WS-14 und WS-30.

### KONSISTENZGEBER UND VERDICKUNGSMITTEL

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### ÜBERFETTUNGSMITTEL UND STABILISATOREN

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Stabilisatoren können Metallsalze von Fettsäuren, wie z.B. Magnesium-, Aluminium- und/oder Zinkstearat bzw. -ricinoleat eingesetzt werden.

### POLYMERE

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

### SILIKONVERBINDUNGEN

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### UV LICHTSCHUTZFAKTOREN

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Üblicherweise sind die UV-Lichtschutzfaktoren in Mengen von 0,1 bis 5 und vorzugsweise 0,2 bis 1 Gew.-% zugegen. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)-benzoe-säureamylester;
- Ester der Zimtsäure, vorzugsweise 4-Methoxyzimtsäure-2-ethylhexylester, 4-Methoxy-zimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexyl-ester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb® HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- 1H-Benzimidazole-4,6-Disulfonic Acid, 2,2'-(1,4-Phenylene)Bis-, Disodium Salt (Neo Heliopan® AP)
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 2-(4-Diethylamino-2-hydroxybenzoyl)-benzoic acid hexylester (Uvinul® A Plus), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans" z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000, Eusolex® T, Eusolex® T-ECO, Eusolex® T-S, Eusolex® T-Aqua, Eusolex® T-45D (alle Merck), Uvinul TiO₂ (BASF). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid wie z.B. Z-COTE® oder Z-COTE HP1® verwendet.

### FEUCHTHALTEMITTEL

Feuchthaltemittel dienen zur weiteren Optimierung der sensorischen Eigenschaften der Zusammensetzung sowie zur Feuchtigkeitsregulierung der Haut. Gleichzeitig wird die Kältestabilität der erfindungsgemäßen Zubereitungen, insbesondere im Falle von Emulsionen, erhöht. Die Feuchthaltemittel sind üblicherweise in einer Menge von 0,1 bis 15 Gew.-%, vorzugsweise 1 bis 10 Gew.-%, und insbesondere 5 bis 10 Gew.-% enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin, Triglycerin und Butylenglycol.

### BIOGENE WIRKSTOFFE UND ANTIOXIDANTIEN

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α -Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### DEODORANTIEN UND KEIMHEMMENDE MITTEL

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

**Keimhemmende Mittel.** Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)harnstoff, 2,4,4'-Trichlor-2'-hydroxydiphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

**Enzyminhibitoren.** Als Enzyminhibitoren sind beispielsweise Esteraseinhibitoren geeignet. Hierbei handelt es sich vorzugsweise um Trialkylcitrate wie Trimethylcitrat, Tripropy-Icitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

**Geruchsabsorber.** Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

**Antitranspirantien.** Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
- adstringierende Wirkstoffe,
- Ölkomponenten,
- nichtionische Emulgatoren,
- Coemulgatoren,
- Konsistenzgeber,
- Hilfsstoffe wie z. B. Verdicker oder Komplexierungsmittel und/oder
- nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichloro-hydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
- entzündungshemmende, hautschützende oder wohlriechende ätherische Öle,
- synthetische hautschützende Wirkstoffe und/oder
- öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### FILMBILDNER

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### ANTISCHUPPENWIRKSTOFFE

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### QUELLMITTEL

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993**)** entnommen werden.

### INSEKTENREPELLENTIEN

Als Insekten-Repellentien kommen N,N-Diethyl-m-toluamid, 1,2-Pentandiol oder Ethyl Butylacetylaminopropionate in Frage. Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### INHALTSSTOFFE FÜR MUND- UND ZAHNPFLEGEMITTEL

Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxid-trihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummiarabicum, Xantham-Gum und Carboxyvinylpolymere (z. B. Carbopol®-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:
- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharin-Natrium, Natrium-Cyclamat, Sucrose, Lactose, Maltose, Fructose oder Apartam®, (L-Aspartyl- L-phenylalanin-methylester), Stiviaextrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;
- wundheilende und entzündungshemmende Stoffe wie z. B. Allantoin, Harnstoff, Azulen, Kamillenwirkstoffe und Acetylsalicylsäurederivate.

Eine bevorzugte Ausführung der kosmetischen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

### HYDROTROPE

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

### KONSERVIERUNGSMITTEL

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### PARFÜMÖLE UND AROMEN

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### FARBSTOFFE

Als Farbstoffe können die für kosmetische Zwecke geeigneten und zugelassenen Substanzen verwendet werden, wie sie beispielsweise in der Publikation **"**Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.1.73015), Chlorophyllin (C.1.75810), Chinolingelb (C.I.47005), Titandioxid (C.1.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### KAUGUMMIS

Bei den bevorzugten oralen Zubereitungen kann es sich auch um (medizinische) Kaugummis handeln. Diese Produkte enthalten typischerweise eine wasserunlösliche und eine wasserlösliche Komponente.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang lkang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500**,** auf deren Inhalt hiermit ausdrücklich Bezug genommen wird.

Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 gew.-% bezogen auf die Kaugummizusammensetzung. Geeignete weitere Geschmacksstoffe stellen beispielsweise essentielle Öle, synthetische Aromen und dergleichen dar, wie Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

### KAPSELN

In einer weiteren Ausführungsform betrifft die Erfindung auch Kapseln, die die oben beschriebenen flüssigen Kühlstoffzubereitungen enthalten.

Die Zubereitungen werden üblicherweise mit Hilfe von festen Überzugsmaterialien verkapselt, wie beispielsweise Stärken, einschließlich deren Abbauprodukten sowie chemisch oder physikalisch erzeugten Derivaten (insbesondere Dextrine und Maltodextrine), Gelatine, Gummi Arabicum, Agar-Agar, Ghatti Gum, Gellan Gum, modifizierte und nicht-modifizierte Cellulosen, Pullulan, Curdlan, Carrageenane, Alginsäure, Alginate, Pektin, Inulin, Xanthan Gum und Mischungen von zwei oder mehreren dieser Substanzen.

Das feste Verkapselungsmaterial ist vorzugsweise eine Gelatine (insbesondere Schweine-, Rind-, Geflügel- und/oder Fischgelatine), wobei diese vorzugsweise einen Schwellfaktor von größer oder gleich 20, vorzugsweise von größer oder gleich 24 aufweist. Ebenfalls bevorzugt sind Maltodextrine (insbesondere auf Basis von Getreide, speziell Mais, Weizen, Tapioka oder Kartoffeln), die vorzugsweise DE-Werte im Bereich von 10 bis 20 aufweisen. Weiterhin bevorzugt sind Cellulosen (z.B. Celluloseether), Alginate (z.B. Natriumalginat), Carrageenan (z.B. beta-, jota-, lambda- und/oder kappa-Carrageenan), Gummi Arabicum, Curdlan und/oder Agar Agar.

Unter diesen Stoffen ist Gelatine besonders bevorzugt, da sie gut verfügbar ist und mit unterschiedlichen Schwellfaktoren bezogen werden kann. Ganz besonders bevorzugt, insbesondere für orale Anwendungen, sind nahtlose Gelatine- oder Alginatkapseln, deren Hülle sich im Mund oder beim Kauen sehr rasch auflöst oder aufbricht. Entsprechende Kapseln werden beispielsweise in den folgenden Schriften ausführlich EP 0389700 A1**,** US 4,251,195**,** US 6,214,376**,** WO 2003 055587 oder WO 2004 050069 A1**.**

Die Kapseln können alternativ auch Mikrokapseln darstellen. Unter den Begriffen "Mikrokapsel" oder "Nanokapsel" werden vom Fachmann sphärische Aggregate mit einem Durchmesser im Bereich von 0,0001 bis 5 und vorzugsweise 0,005 bis 0,5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Nach einem dritten Verfahren werden Partikel abwechselnd mit Polyelektrolyten unterschiedlicher Ladung beschichtet ("layer-by-layer"-Verfahren). Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide).

Chitosanmikrokapseln und Verfahren zu ihrer Herstellung sind aus dem Stand der Technik hinlänglich bekannt **[**WO 01/01926**,** WO 01/01927**,** WO 01/01928**,** WO 01/01929**].** Mikrokapseln mit mittleren Durchmessern im Bereich von 0,0001 bis 5, vorzugsweise 0,001 bis 0,5 und insbesondere 0,005 bis 0,1 mm, bestehend aus einer Hüllmembran und einer die Wirkstoffe enthaltenden Matrix, können beispielsweise erhalten werden, indem man
(a) aus Gelbildnern, kationischen Polymeren und Wirkstoffen eine Matrix zubereitet,
(b) gegebenenfalls die Matrix in einer Ölphase dispergiert,
(c) die dispergierte Matrix mit wässrigen Lösungen anionischer Polymere behandelt und gegebenenfalls dabei die Ölphase entfernt.

Die Schritte (a) und (c) sind dabei insofern austauschbar, als man anstelle der kationischen Polymeren in Schritt (a) anionische Polymere einsetzt und umgekehrt.

Man kann die Kapseln auch erzeugen, indem man den Wirkstoff abwechselnd mit Schichten aus unterschiedlich geladenen Polyelektrolyten einhüllt (layer-by-layer-Technologie). In diesem Zusammenhang sei auf das Europäische Patent EP 1064088 B1 (Max-Planck Gesellschaft) verwiesen.

### GEWERBLICHE ANWENDBARKEIT

Ein weiterer Gegenstand der vorliegenden Erfindung betrifft ein nicht-therapeutisches Verfahren zur Kühlung und Beruhigung von Haut oder Schleimhäuten, bei dem man diese mit einer Menge der erfindungsgemäßen flüssigen Kühlstoffzubereitungen in Kontakt bringt. Dies kann insbesondere dadurch erfolgen, dass eine der vorstehend erläuterten kosmetischen Zubereitungen topisch aufgetragen wird.

Schließlich betrifft die Erfindung auch flüssige Kühlstoffzubereitungen zur Verwendung bei der Herstellung von dermatologischen und/oder pharmazeutischen Zubereitungen in der oben ausführlich beschriebenen Art und Weise.

### BEISPIELE

### BEISPIELE 1 BIS 4, VERGLEICHSBEISPIEL V1

Verschiedene Kühlstoffmischungen wurden hergestellt und bei 5 bzw. 20 °C gelagert. Die Zusammensetzung der Mischungen und ihr Lagerverhalten ist in **Tabelle 1** wiedergegeben:

**Tabelle 1**

| Lagerverhalten von Kühlstoffmischungen | | | | | |
|---|---|---|---|---|---|
| **Komponente** | **1** | **2** | **3** | **4** | **V1** |
| L-Menthol | 52,00 | 52,00 | 38.00 | 38,00 | 50,00 |
| D-Menthol | 28.00 | 28,00 | 38,00 | 38,00 | 50,00 |
| Iso-Menthol | - | - | 4,00 | 4,00 | - |
| Menthylacetat (L) | 20,00 | 9,50 | 20,00 | 9,50 | - |
| Menthylacetat (D) | - | 9,50 | - | 9,50 | - |
| Menthylacetat (iso) | - | 0,60 | - | 0,60 | - |
| Essigsäurementhyllactat | - | 0,40 | - | 0,40 | - |

| ***Lagerverhalten*** | | | | | |
|---|---|---|---|---|---|
| Sofort | flüssig | flüssig | flüssig | flüssig | fest |
| 1 Tag, 20 °C | flüssig | flüssig | flüssig | flüssig | fest |
| 1 Tag, 5 °C | flüssig | flüssig | flüssig | flüssig | fest |
| 5 Tage, 20 °C | flüssig | flüssig | flüssig | flüssig | fest |

Die Beispiele und Vergleichsbeispiele zeigen die Überlegenheit der erfindungsgemäßen Zubereitungen, die sowohl in der Kälte als auch bei Umgebungstemperatur flüssig vorlagen, während die Mischung aus den beiden Mentholisomeren fest ist. Die neuen Gemische erwiesen sich zudem in Geschmack, Geruch und Kühlvermögen Menthol als völlig gleichwertig.

## Patentansprüche

1. Kühlstoffzubereitungen, enthaltend
(a) L-Menthol,
(b) D-Menthol und
(c) mindestens einen weiteren Kühlstoff, ausgewählt aus der Gruppe, die gebildet wird von Menthylacetat (L), Menthylacetat (D), Menthylacetat (iso), Essigsäurementhyllactat (iso) und Essigsäurementhyllactat (neo),
mit der Maßgabe, dass diese im Temperaturbereich von 5 bis 20 °C flüssig vorliegen.

2. Zubereitungen nach Anspruch 1, **dadurch gekennzeichnet, dass** sie
(a) 35 bis 55 Gew.-% L-Menthol,
(b) 25 bis 40 Gew.-% D-Menthol und
(c) 15 bis 30 Gew.-% Menthylacetat (L), Menthylacetat (D), Menthylacetat (iso), Essigsäurementhyllactat (iso) und Essigsäurementhyllactat (neo) oder deren Gemische
mit der Maßgabe enthalten, dass sich die Mengenangaben jeweils zu 100 Gew.-% ergänzen.

3. Zubereitungen nach Anspruch 1, **dadurch gekennzeichnet, dass** sie
(a) L-Menthol,
(b) D-Menthol und
(c) Menthylacetat (L)
enthalten.

4. Zubereitungen nach Anspruch 3, **dadurch gekennzeichnet, dass** sie
(a) 50 bis 55 Gew.-% L-Menthol,
(b) 25 bis 35 Gew.-% D-Menthol und
(c) 18 bis 23 Gew.-% Menthylacetat (L)
mit der Maßgabe enthalten, dass sich die Mengenangaben jeweils zu 100 Gew.-% ergänzen.

5. Zubereitungen nach Anspruch 1, **dadurch gekennzeichnet, dass** sie
(a) L-Menthol,
(b) D-Menthol,
(c1) Menthylacetat (L),
(c2) Menthylacetat (D),
(c3) Menthylacetat (iso) und
(c4) Essigsäurementhyllactat (neo, iso)
enthalten.

6. Zubereitungen nach Anspruch 5, **dadurch gekennzeichnet, dass** sie
(a) 50 bis 55 Gew.-% L-Menthol,
(b) 25 bis 35 Gew.-% D-Menthol,
(c1) 8 bis 12 Gew.-% Menthylacetat (L),
(c2) 8 bis 12 Gew.-% Menthylacetat (D),
(c3) 0,1 bis 1 Gew.-% Menthylacetat (iso) und
(c4) 0,1 bis 1 Gew.-% Essigsäurementhyllactat (neo, iso)
mit der Maßgabe enthalten, dass sich die Mengenangaben jeweils zu 100 Gew.-% ergänzen.

7. Zubereitungen enthaltend
(a) L-Menthol,
(b) D-Menthol,
(c1) iso-Menthol und
(c2) Menthylacetat (L).

8. Zubereitungen nach Anspruch 7, **dadurch gekennzeichnet, dass** sie
(a) 35 bis 45 Gew.-% L-Menthol,
(b) 35 bis 45 Gew.-% D-Menthol,
(c1) 2 bis 8 Gew.-% iso-Menthol und
(c2) 15 bis 25 Gew.-% Menthylacetat (L)
mit der Maßgabe enthalten, dass sich die Mengenangaben jeweils zu 100 Gew.-% ergänzen.

9. Zubereitungen enthaltend
(a) L-Menthol,
(b) D-Menthol,
(c1) iso-Menthol,
(c2) Menthylacetat (L),
(c3) Menthylacetat (D),
(c4) Menthylacetat (iso) und
(c5) Essigsäurementhyllactat (neo, iso).

10. Zubereitungen nach Anspruch 9, **dadurch gekennzeichnet, dass** sie
(a) 35 bis 45 Gew.-% L-Menthol,
(b) 35 bis 45 Gew.-% D-Menthol,
(c1) 2 bis 8 Gew.-% iso-Menthol,
(c2) 8 bis 12 Gew.-% Menthylacetat (L),
(c3) 8 bis 12 Gew.-% Menthylacetat (D),
(c4) 0,1 bis 1 Gew.-% Menthylacetat (iso) und
(c5) 0,1 bis 1 Gew.-% Essigsäurementhyllactat (neo, iso)
mit der Maßgabe enthalten, dass sich die Mengenangaben jeweils zu 100 Gew.-% ergänzen.

11. Kosmetische und/oder dermatologische und/oder pharmazeutische Zubereitungen enthaltend die flüssigen Kühlstoffzubereitungen nach Anspruch 1.

12. Zubereitungen nach Anspruch 11, **dadurch gekennzeichnet, dass** sie die flüssigen Kühlstoffzubereitungen in Mengen von 0,1 bis 10 Gew.-% enthalten.

13. Kapseln, enthaltend die flüssigen Kühlstoffzubereitungen nach Anspruch 1.

14. Nicht-therapeutisches Verfahren zur Kühlung und Beruhigung von Haut oder Schleimhäuten, bei dem man diese mit einer Menge der flüssigen Kühlstoffzubereitungen nach Anspruch 1 in Kontakt bringt.

15. Flüssige Kühlstoffzubereitungen nach Anspruch 1 zur Verwendung bei der Herstellung von dermatologischen und/oder pharmazeutischen Zubereitungen.

## Claims

1. Cooling preparations containing
(a) L-menthol,
(b) D-menthol and
(c) at least one further cooling agent selected from the group consisting of menthyl acetate (L), menthyl acetate (D), menthyl acetate (iso), acetic acid menthyl lactate (iso) and acetic acid menthyl lactate (neo),
with the proviso that they are liquid in the temperature range of 5 to 20 °C.

2. The preparations according to claim 1, **characterized in that** they contain
(a) 35 to 55 % by weight of L-menthol,
(b) 25 to 40% by weight of D-menthol and
(c) 15 to 30 % by weight of menthyl acetate (L), menthyl acetate (D), menthyl acetate (iso), menthyl acetate of acetic acid (iso) and menthyl acetate of acetic acid (neo) or mixtures thereof
with the proviso that the quantities indicated in each case add up to 100% by weight.

3. The preparations according to claim 1, **characterized in that** they contain
(a) L-menthol,
(b) D-menthol and
(c) menthyl acetate (L).

4. The preparations according to claim 3, **characterized in that** they
(a) 50 to 55 % by weight of L-menthol,
(b) 25 to 35 % by weight of D-menthol and
(c) 18 to 23 % by weight of menthyl acetate (L)
with the proviso that the quantities indicated in each case add up to 100% by weight.

5. The preparations according to claim 1, **characterized in that** they contain
(a) L-menthol,
(b) D-menthol,
(c1) menthyl acetate (L),
(c2) menthyl acetate (D),
(c3) menthyl acetate (iso) and
(c4) acetic acid menthyl lactate (neo, iso).

6. The preparations according to claim 5, **characterized in that** they contain
(a) 50 to 55 % by weight of L-menthol,
(b) 25 to 35 % by weight of D-menthol,
(c1) 8 to 12 % by weight of menthyl acetate (L),
(c2) 8 to 12 % by weight of menthyl acetate (D),
(c3) 0.1 to 1% by weight of menthyl acetate (iso) and
(c4) 0,1 to 1 % by weight of acetic acid menthyl lactate (neo, iso)
with the proviso that the quantities indicated in each case add up to 100% by weight.

7. Preparations containing
(a) L-menthol,
(b) D-menthol,
(c1) iso-menthol and
(c2) menthyl acetate (L).

8. The preparations according to claim 7, **characterized in that** they contain
(a) 35 to 45 % by weight of L-menthol,
(b) 35 to 45 % by weight of D-menthol,
(c1) 2 to 8% by weight of iso-menthol and
(c2) 15 to 25 % by weight of menthyl acetate (L)
with the proviso that the quantities indicated in each case add up to 100% by weight.

9. The preparations containing
(a) L-menthol,
(b) D-menthol,
(c1) iso-menthol,
(c2) menthyl acetate (L),
(c3) menthyl acetate (D),
(c4) menthyl acetate (iso) and
(c5) acetic acid menthyl lactate (neo, iso).

10. The preparations according to claim 9, **characterized in that** they contain
(a) 35 to 45 % by weight of L-menthol,
(b) 35 to 45 % by weight of D-menthol,
(c1) 2 to 8% by weight of iso-menthol,
(c2) 8 to 12 % by weight of menthyl acetate (L),
(c3) 8 to 12 % by weight of menthyl acetate (D),
(c4) 0.1 to 1% by weight of menthyl acetate (iso) and
(c5) 0,1 to 1 % by weight of acetic acid menthyl lactate (neo, iso)
with the proviso that the quantities indicated in each case add up to 100% by weight.

11. Cosmetic and/or dermatological and/or pharmaceutical preparations containing the liquid coolant preparations according to claim 1.

12. Preparations according to claim 11, **characterized in that** they contain the liquid coolant preparations in amounts of 0.1 to 10% by weight.

13. Capsules containing the liquid coolant preparations according to claim 1.

14. A non-therapeutic process for cooling and soothing skin or mucous membranes, in which they are brought into contact with an amount of the liquid coolant preparations according to claim 1.

15. The liquid coolant preparations according to claim 1 for use in the manufacture of dermatological and/or pharmaceutical preparations.

## Revendications

1. Préparations réfrigérantes contenant
(a) L-menthol,
(b) le D-menthol et
(c) au moins un autre agent de refroidissement choisi dans le groupe constitué par l'acétate de menthyle (L), l'acétate de menthyle (D), l'acétate de menthyle (iso), le lactate de menthyle d'acide acétique (iso) et le lactate de menthyle d'acide acétique (néo),
à condition qu'ils soient liquides dans la plage de température de 5 à 20 °C.

2. Les préparations selon la revendication 1, **caractérisées en ce qu'**elles contenu
(a) 35 à 55 % en poids de L-menthol,
(b) 25 à 40 % en poids de D-menthol et
(c) 15 à 30 % en poids d'acétate de menthyle (L), d'acétate de menthyle (D), d'acétate de menthyle (iso), d'acétate de menthyle (iso) et d'acétate de menthyle (néo) ou de leurs mélanges
à condition que les quantités indiquées se complètent à 100 % en poids.

3. Les préparations selon la revendication 1, **caractérisées en ce qu'**elles contenu
(a) L-menthol,
(b) le D-menthol et
(c) acétate de menthyle (L).

4. Les préparations selon la revendication 3, **caractérisées en ce qu'**elles contenu
(a) 50 à 55 % en poids de L-menthol,
(b) 25 à 35 % en poids de D-menthol et
(c) 18 à 23 % en poids d'acétate de menthyle (L)
à condition que les quantités indiquées se complètent à 100 % en poids.

5. Les préparations selon la revendication 1, **caractérisées en ce qu'**elles contenu
(a) L-menthol,
(b) D-menthol,
(c1) acétate de menthyle (L),
(c2) acétate de menthyle (D),
(c3) acétate de menthyle (iso) et
(c4) acide acétique menthyl lactate (neo, iso).

6. Les préparations selon la revendication 5, **caractérisées en ce qu'**elles contenu
(a) 50 à 55 % en poids de L-menthol,
(b) 25 à 35 % en poids de D-menthol,
(c1) 8 à 12 % en poids d'acétate de menthyle (L),
(c2) 8 à 12 % en poids d'acétate de menthyle (D),
(c3) 0,1 à 1 % en poids d'acétate de menthyle (iso) et
(c4) 0,1 à 1 % en poids d'acide acétique lactate de menthyle (néo, iso)
à condition que les quantités indiquées se complètent à 100 % en poids.

7. Les préparations contenant
(a) L-menthol,
(b) D-menthol,
(c1) l'isomenthol et
(c2) acétate de menthyle (L).

8. Les préparations selon la revendication 7, **caractérisées en ce qu'**elles contenu
(a) 35 à 45 % en poids de L-menthol,
(b) 35 à 45 % en poids de D-menthol,
(c1) 2 à 8 % en poids d'isomenthol et
(c2) 15 à 25 % en poids d'acétate de menthyle (L)
à condition que les quantités indiquées se complètent à 100 % en poids.

9. Les préparations contenant
(a) L-menthol,
(b) D-menthol,
(c1) l'isomenthol,
(c2) acétate de menthyle (L),
(c3) acétate de menthyle (D),
(c4) acétate de menthyle (iso) et
(c5) acide acétique menthyl lactate (neo, iso).

10. Les préparations selon la revendication 9, **caractérisées en ce qu'**elles contenu
(a) 35 à 45 % en poids de L-menthol,
(b) 35 à 45 % en poids de D-menthol,
(c1) 2 à 8 % en poids d'isomenthol,
(c2) 8 à 12 % en poids d'acétate de menthyle (L),
(c3) 8 à 12 % en poids d'acétate de menthyle (D),
(c4) 0,1 à 1 % en poids d'acétate de menthyle (iso) et
(c5) 0,1 à 1 % en poids d'acide acétique lactate de menthyle (néo, iso)
à condition que les quantités indiquées se complètent à 100 % en poids.

11. Les préparations cosmétiques et/ou dermatologiques et/ou pharmaceutiques contenant les préparations réfrigérantes liquides selon la revendication 1.

12. Les préparations selon la revendication 11, **caractérisées en ce qu'**elles contiennent les préparations de réfrigérant liquide dans des quantités de 0,1 à 10 % en poids.

13. Capsules contenant les préparations de liquide de refroidissement selon la revendication 1.

14. Méthode non thérapeutique pour refroidir et apaiser la peau ou les muqueuses, dans laquelle ils sont mis en contact avec une quantité de préparations liquides de refroidissement selon la revendication 1.

15. Les préparations liquides de refroidissement selon la revendication 1, destinées à être utilisées dans la fabrication de préparations dermatologiques et/ou pharmaceutiques.
